# EUROPEAN PATENT SPECIFICATION

(11) **EP 1 848 447 B1**
(45) Date of publication and mention of the grant of the patent: **12.11.2014**
(21) Application number: 05790886.5
(22) Date of filing: 19.08.2005
(51) Int. Cl.: A61K 35/14, A61K 35/16

(54) **BIOLOGICAL TISSUE REGENERATIVE AGENT AND METHOD FOR PREPARING AND USING SAME**
MITTEL ZUR REGENERATION VON BIOLOGISCHEM GEWEBE UND VERFAHREN ZU SEINER HERSTELLUNG UND VERWENDUNG
AGENT DE REGENERATION D'UN TISSU BIOLOGIQUE ET METHODE DE PREPARATION ET D'UTILISATION

(30) Priority: 20.08.2004 US 923237
(43) Date of publication of application: 31.10.2007
(73) Proprietor: Gorrochategui Barrueta, Alberto, 48010 Bilbao Vizcaya (ES)
(72) Inventor: Gorrochategui Barrueta, Alberto, 48010 Bilbao (ES); Simon Elizundia, Josu, 48010 Bilbao (ES); Barbera-Guillem, Emilio, Powell, OH 43065 (US)
(74) Representative: ABG Patentes, S.L.
(86) International application number: PCT/US2005/029950
(87) International publication number: WO 2006/023911

(56) References cited:
- WO-A-95/15763
- US-A- 5 165 938
- US-A- 5 165 938
- US-A- 5 578 326
- US-A1- 2003 007 957
- US-A1- 2004 151 709

## Description

### TECHNICAL FIELD

The present invention relates to the field of biological tissue growth and repair, particularly to a regenerative agent that enhances tissue and cell growth, repair, and transplantation and a method for preparing and using same.

### BACKGROUND OF THE INVENTION

Traumatic or pathological tissue injury leads to loss of biological tissue, followed by natural attempts at repair and tissue remodeling. Due to attendant failures in natural repair, human and veterinary medicine attempts to intervene in the natural repair and remodeling process via a number of modalities. This can include the implantation of differentiated cells in the form of partial or entire organ transplants, however, this modality is limited by the supply of compatible tissues, problems of rejection, and technical difficulties in transplantation. More recently, attention has been drawn to the possibilities of implanting multipotent or pluripotent stem cells for tissue regeneration. All these modalities of exogenous cellular introduction are affected by what may be called the interface problem, in that they are all plagued by problems of slow angiogenesis, inflammation, potential infection, and immune rejection issues. Even entirely natural tissue regeneration is susceptible to some of these problems, resulting in extended healing time and even less than optimal healing and tissue regeneration.

A stimulant to both the natural and exogenously assisted tissue regeneration process is the provision of various elements that accelerate or otherwise assist in the regenerative process. On the simplest level, an example is the provision of natural and artificial matrices, such as bone grafts; or the use of natural and artificial tissue bonding agents, such as the use of fibrin clot or various artificial tissue adhesives. Such materials provide a physical matrix for new cells to grow upon or hold tissues in proximity during the natural healing process.

Alternatively, various biological stimulants to cellular regrowth have been proposed. These include angiogenic factors such as vascular endothelial growth factor (VEGF) and platelet derived growth factor (PDGF) from both natural preparations and recombinant sources; and tissue growth factors, such as epidermal growth factor (EGF), again from both natural preparations and recombinant sources. They also include immune modulators, which can include, among others; recombinant transforming growth factor (TGF-β); the application of autologous plasma or serum preparations that include natural immune modulators; or drugs, such as cyclosporine A. Increasingly, attention is being drawn to so-called synergistic factors, a poorly understood group including such factors as antioxidants, that are believed to assist in the regeneration and integration of new tissues.

All of these compounds are known, or believed, to exist in considerable amounts in platelets. The contribution of various platelet derived factors to biological tissue repair is well known. Platelets are the smallest corpuscular components of human blood (diameter 2-4µm), and their physiological number varies from 150,000 to 300,000/mm³ in normal human blood. Platelets are not true cells, but are essentially cell fragments formed without nuclear material. The origin of platelets is the megakaryocyte, a hematopoietic cell normally resident in the bone marrow. The megakaryocytes are giant cells generated by both the mitotic growth of a committed progenitor cell, and the endoreduplication of genetic material of the cell without cell division. This last modality of cell growth yields cells with polyploid nuclei and a large cytoplasm, in which the internal membranes, through a process of division into plots, generates the proplatelets or platelet territories. The mature megakaryocyte, with the cytoplasm segmented into platelet territories, gives off platelets as the end product of protrusions of their cytoplasm through the capillary walls. Platelets contain a variety of internal organelles, such as mitochondria that supply energy, golgi bodies, and glycogen particles; and a microtubular and microfibrillar cytoskeletal system; but one of their most studied features is a system of α-granules and dense bodies that constitute the main storage compartments of the platelets.

While various plasma preparations including whole or fragmented platelets have been used to stimulate tissue regeneration, the presence of histocompatibilty sites on platelet membranes and the resulting immunocompatibility issues has directed attention to purified platelet preparations, especially those of compounds released from intact platelets during platelet activation.

A platelet in circulation possesses about 35 α-granules and 5 dense bodies as main storage compartments. The α-granules contain an array of peptide mediators; including the chemokines platelet factor-4 (PF-4), *b*-thromboglobulin (*b*-TG), regulated upon activation normal T cell expressed and secreted (RANTES) chemokines, and macrophage inflammatory protein-1a (MIP-1a).

PF-4 and b-TG are first-line mediators in recruitment and activation of leukocytes. PF-4 displays chemotaxis for neutrophils, monocytes, and fibroblasts. It induces the release of histamine from basophils and stimulates adherence of eosinophils. Furthermore, PF-4 prevents apoptosis of monocytes and initiates their differentiation into macrophages. Neutrophils are induced by PF-4 to adhere to unstimulated endothelium and to release the content of their secondary granules. The *b*-TG proteins, which are proteolytic products of inactive precursors, may act as stimulatory or inhibitory agents in neutrophil activation, depending on their processing.

The chemokine RANTES induces the release of histamine from basophils and secretion of cationic proteins from eosinophils. Moreover, RANTES contributes to inflammatory or atherogenic recruitment of monocytes from the circulation as it is deposited on activated microvascular and arterial endothelium and triggers shear-resistant monocyte arrest. MIP-1*a* also has histamine-releasing activity on basophils and displays chemotaxis for CD81 T lymphocytes.

In turn, chemokines, such as RANTES and MIP-1*a*, stimulate platelets to give Ca21 signals, to aggregate, and to release their granular contents. Both functional, that is, CCR1, CCR3, and CCR4; and CXC (CXCR4) chemokine receptors are detectable on human platelets.

The demonstration of several growth factors in α-granules has been particularly important. Platelets store large amounts of vascular endothelial growth factor (VEGF), which is released on stimulation by thrombin and aggregation. VEGF promotes extravasation of plasma proteins, thereby guiding the migration of leukocytes and endothelial cells and causing edema. VEGF also supports wound fluid accumulation and initiates angiogenesis in the wound repair stage. PDGF is chemotactic for neutrophils and monocytes, and it controls recruitment and proliferation of fibroblasts and smooth muscle cells. TGF-β influences the wound healing in a bidirectional manner from the earliest steps in inflammation to the final deposition and remodeling of the extracellular matrix (ECM).

TGF-β elicits the rapid chemotaxis and activation of neutrophils and monocytes, but, later in the process, suppresses the inflammatory response. Fibroblast growth factors (FGFs) signal chemotaxis and mitogenesis. They recruit endothelial cells and direct their proliferation, thus playing a central role in angiogenesis together with angiopoietin, which also is released from stimulated platelets and stabilizes proliferating endothelial cells. Epidermal growth factor (EGF) contributes to recruitment and growth of fibroblasts and epithelial cells in the formation of granulation tissue.

Platelets play a central regulatory role in all stages of inflammation and subsequent wound repair by the release, upon stimulation, of all these factors from their α-granules. In contrast to the α-granular agents, the mediators released from the dense bodies exert effects that are more restricted to the initial phase of inflammation. Adenosine disphosphate (ADP) augments the agonist induced oxidative burst in neutrophils. Serotonin increases vascular permeability. Histamine is also involved in the regulation of endothelial permeability, and enhances the production of superoxides by macrophages. The importance of dense body-derived serotonin is underlined by its key role in hypersensitivity reactions of the skin.

In addition to their capacity to release prestored mediators, stimulated platelets are able to generate eicosanoids for regulation of hemostasis and acute inflammation. Platelet products of cycloxygenase-dependent arachidonic acid metabolism are thromboxane A2 (TXA2), prostaglandin F2*a* (PGF2*a*), and PGE2. TXA2 and PGF2*a* cause vasoconstriction. PGE2 is a vasodilator and modulator of pain. 12-Hydroxyeicosatetraenoic acid (12-HETE), synthesized by a platelet-specific enzyme, is chemotactic toward eosinophils. Recent studies indicate that platelets can synthesize peptide mediators, such as IL-1*b*, on stimulation. Protein synthesis in platelets is controlled via integrin *b*3-mediated translocation of eukaryotic initiation factor 4E to the cytoskeleton. Thus, platelets are able to respond to stimulation not only by synthesis of eicosanoids, nitric oxide, and reactive oxygen species, but also by regulated *de novo* synthesis of peptide mediators.

Knighton *et al.,* described angiogenesis and platelet derived growth factors in "Role of Platelets and Fibrin in the Healing Sequence," Annals of Surgery; 196:379-388 (1982). Of particular interest has been the role of PDGF, which is known to be comprised of two polypeptides, PDGF-I and PDGF-II, as described by Grotendorst et al., in "Platelet Derived Growth Factor is a Chemoattractant for Vascular Smooth Muscle Cells," Journal of Cellular Physiology; 113:261-66 (1982). In U.S. Pat. No. 4,479,896 ('896) to Antoniades, a method of isolating PDGF from lysed platelets in aqueous solution is reported. Gel electrophoresis is used to isolate purified PDGF-I and PDGF-II, which is recovered by elution. A final recovery of 3.7% of the PDGF-I and 2.0% of the PDGF-II was reported.

It is also well known that thrombin causes the platelet release reaction in suspensions of washed platelets. Tollefsen et al., "Induction of the Platelet Release Reaction by Phytohemagglutinin," J. Clin. Invest.; 53:211-218 (1974). Thrombin is a potent agonist that elicits platelet physiological responses such as shape change, granular content secretion, and aggregation. Thrombin is a multifunctional serine protease that acts as primary agonist for the platelet release reaction. Goldsack, et al., "Molecules in Focus - Thrombin," Int. J. Bioch. and Cell Biol.; 30:641-46 (1998). Thrombin's effect on human platelets is thought to be primarily mediated through two protease activated receptors: PAR1 and PAR4. Ofosu, F, "Protease Activated Receptors 1 and 4 Govern the Responses of Human Platelets to Thrombin," Transfusion and Apherisis Science 28:265 (2003).

Cleavage of PAR1 occurs at Arg41-Ser42 located within the long exodomain of the receptor. SFLLRN is a new amino-terminus and functions as a tethered peptide ligand binding another site on the thrombin receptor and induces an intracellular signaling cascade. Santos, B. et al., "Interaction of Viper Venom Serine Peptidases with Thrombin Receptors on Human Platelets," Fed. of Eur. Bioch. Soc. Letters 477:199 (2000). Responses of platelets to thrombin include cytokinesis, aggregation, and secretion, as well as associated metabolic changes.

Although anuclear, platelets are very reactive bodies that contain all of the exocytotic machinery involved in cellular secretion. The platelet release reaction resembles other regulated secretion events. Unlike platelet lysis, which involves a complete disruption of the platelet membrane and a release of all intra-platelet compounds, platelet release is a coordinated and tightly regulated secretion that consists of: 1) movement of the granules towards the plasma membrane due to microtubule and granule-bound GTP-binding proteins, 2) docking, and 3) fusion with the membrane with the consequent unloading of the granules' constituents. Rendu, F. et al., "The Platelet Release Reaction: Granules' Constituents, Secretion and Functions," Platelets 12:261-73 (2001). Thrombin plays a vital role in blood coagulation by promoting platelet aggregation and by converting fibrinogen to form the fibrin clot in the final step of the coagulation cascade.

Knighton, as noted above, teaches a method of using thrombin, adenosine disphosphate, or collagen to trigger the platelet release reaction and subsequent degranulation of platelets in order to produce a composition for treating damaged tissue in U.S. Pat. No. 5,165,938 ('938). In the Knighton process, blood is anticoagulated with a 20% citrate-phosphate-dextrose solution to competitively bind extracellular calcium and thereby to prevent clotting. The blood is centrifuged to yield a platelet rich plasma of preferably at least 1,000,000 platelets per ml. The platelet free plasma is removed and discarded and the platelet pellet, resulting from centrifugation, is resuspended in a platelet buffer containing HEPES (N-2-hydroxyethylpiperazine-n-2-ethanesulfonic acid), glucose, KCl, NaCl, and about 0.35% human serum albumin, in a highly acid (pH about 6.5) environment.

The resuspended platelets are activated with purified thrombin derived from either bovine or pig thrombin, and allowed to incubate for about 5-10 minutes. The activity of the thrombin coagulates the fibrinogen and activates platelets causing them to release α-granules containing platelet-derived growth factor and platelet-derived angiogenesis factor. The thrombin derived from bovine blood, such as that used in the Knighton process, is commonly used to effect hemostasis in surgeries in the United States and the world. Unfortunately, recent reports suggest that bovine thrombin's use may pose previously unappreciated risks, such as the development of autoreactive antibodies, at least some of which are pathogenic. For example, a recent study utilizing nonautoimune-prone galactose-α1-3-galactose-deficient mice treated with two bovine preparations currently available in the U.S., found that those mice developed an immune response against the foreign thrombin, and some developed autoantibodies against clotting factors. Further, a single exposure induced autoimmunity with features characteristic of systemic lupus erythematosus. Schoenecker, J. et al, "Exposure of Mice to Topical Bovine Thrombin Induces Systemic Autoimmunity," Am. J. Pathol.; 159:1957-1969 (2001). Accordingly, avoidance of animal thrombin in human medicine, when possible, may be prudent.

Additionally, the instant invention provides an increase in both the amount and types of proteins liberated from the platelets. The function of the platelet is primary hemostasis-the formation of a platelet plug. This is initiated by adhesion of the platelets to an area of injury. The platelets adhere to the site of injury, in response to contact with collagen and vonWillebrand factor in the underlying tissue, become activated, and release the alpha granules' contents. These activated platelets aggregate, and are attracted to each other, sticking together to form a soft mass. After adhesion, viscous metamorphosis occurs and the cell membranes dissolve, as a fragile jellylike plug is formed. It is well known that the products of platelet lysis differ from those of the platelet release reaction, due to the additional release of cytosolic components in lysis. Marcus, K. et al., "Identification of Platelet Proteins Separated by Two-Dimensional Gel Electrophoresis and Analyzed by Matrix Assisted Laser Desorption/Ionization-Time of Flight-Mass Spectrometry and Detection of Tyrosine-Phosphorylated Proteins," Electrophoresis 21(13):2622-36 (2000).

When cells undergo the platelet release reaction, compounds are discharged directly from specific granules, such as dense granules, α-granules, and lysosomes, within the cells; but the platelet membrane remains intact and the granules themselves are not discharged. Kinlough-Rathbone, R. et al., "Conditions Influencing Platelet Lysis," Lab. Invest. 32:3 352-358 (1975). Kinlough *et al.* showed two main points with regard to platelet lysis: 1) thrombin treatment alone did not lead to lysis in human platelets, as measured by LDH (lactate dehydrogenase), a marker for a cytoplasmic enzyme that is only released upon lysis; and 2) calcium in the platelet suspending medium is necessary for autolysis of the platelets in the rabbit. Therefore one would expect, and the instant invention shows, that platelet lysis mediated by calcium results in a different array and concentration of physiological compounds than does the platelet release reaction mediated by thrombin. Equally importantly, the avoidance of a platelet release reaction, and the provision of alpha-2 macroglobulin in the serum of the instant invention, suppresses the activation of TGF-β, compared to the array of compounds produced by the platelet release reaction described above. The composition comprising a platelet-serum extract disclosed in WO 95/15763 has TGF-β in an active form, and US 2004/151709 and US 2003/007957 disclose a composition comprising a platelet extract in the absence of serum containing alpha-2 macroglobulin. Interestingly, when platelets take part in the formation of hemostatic plugs and thrombi in vivo, electron microscopic evidence indicates that some of the platelets not only release their granule contents but also undergo lysis. *Id*. This finding suggests that platelet lysis is an important aspect in *in vivo* clot formation.

It is possible to capture platelets' full complement of regenerative factors, and avoid activation of TGF-β, when they undergo lysis. These factors include cytoplasmic enzymes and other cytoplasmic constituents. *Id.* Components that have been identified as being contained in the platelet cytosol include: matrix metalloproteinase-2 (MMP-2), phospholipase A2, phosphatidylinositol-specific phospholipase C, calmodulin, pp60c-src (activates platelet-activating factor (PAF)), cyclic nucleotide phosphodiesterase, cyclic GMP-stimulated cyclic nucleotide phosphodiesterase, c21KG, most enzymes of glycolysis, enzymes of the hexose monophosphate shunt, phosphoglucose isomerase, glutathione reductase, about half of the total platelet adenosine tri-phosphate and adenosine di-phosphate, most glycolytic intermediates (i.e., fructose 1,6-disphosphate), glycogen, phenol sulfotransferase, sulfotransferase, inositol polyphosphate 4-phosphatase, phosphatidylinositol 3-kinase, Gbetagamma-responsive phosphoinositide 3-kinases, Rho-dependent phosphoinositide 3-kinase, G-protein beta gamma-subunit-responsive phosphoinositide 3-kinase, GTP-binding protein (G protein), and free arachidonic acid (AA). Lysis of platelets has been reported with a number of *in vitro* techniques, including sonication, exposing them to repeated freeze-thaw cycles, and by the use of detergent. Kamath, S. et al., "Platelet P-Selectin Levels in Relation to Plasma Soluble P-Selectin and β-Thromboglobulin Levels in Atrial Fibrillation," Stroke 33:1237-1242 (2002). Platelets may also be lysed by increasing, *in vitro,* the extracellular calcium to induce viscous metamorphosis followed by lysis. Calcium induced lysis can be easily distinguished from the platelet release reaction as observed by light-microscopy. Platelets at rest are biconvex discs with a large diameter of about 3 µm. In response to an activating stimulus such as thrombin, platelets lose their discoidal shape and develop long pseudopods within a few seconds. This shape change is accompanied by the secretion of a wealth of adhesive and proinflammatory substances-the so-called release reaction. Klinger, M, "Platelets and Inflammation," Anat. Embryol. 196:1-11 (1997). In contrast, when platelet rich plasma is placed in a high calcium solution, a process known as viscous metamorphosis is initiated in the platelets. During this process the tubulin polymers are reduced to monomers, the actin and myosin networks are disrupted and the cell changes from discoid to a swollen sphere shape. Ultimately the process ends in lysis and the granular and cytoplasmic constituents are released into the surrounding milieu. The instant invention seeks to take advantage of the wealth of compounds that are not released, or are incompletely released, via the platelet release reaction as seen in processes such as the '938 method.

In addition to the effects on tissue healing and growth effected by platelet related compounds, it is known that plasma contains components which are also important to wound repair. Plasma, a yellow liquid, is composed mainly of lightly salted water. Its physiochemical properties are remarkably constant, especially its pH, which is physiologically maintained at pH 7.42, and the concentration of various inorganic elements. Besides being a medium for the flow of blood cells, plasma is the straw-colored liquid in which the blood cells are suspended and has an approximate composition of: Water~92%, Proteins 6-8%, Salts 0.8%, Lipids 0.6%, and Glucose 0.1%. Plasma transports materials needed by cells and materials that must be removed from cells. These include: a) various ions, such as, Na⁺, Ca²⁺, and HCO₃⁻; b) glucose and traces of other sugars; c) amino acids; d) other organic acids; e) cholesterol and other lipids; f) hormones and growth factors; g) urea and other wastes, and numerous other compounds.

Most of these materials are in transit from a place where they are added to the blood (a "source"); to places ("sinks") where they will be removed from the blood, including exchange organs such as the intestine or kidney, and depots of materials such as the liver. Plasma proteins make up 6-8% of the plasma. Researchers have identified more than 4,000 distinctive proteins in human blood plasma.

After blood is withdrawn from a vein and allowed to clot, the clot slowly shrinks. As it does so, a clear fluid called serum is squeezed out. Thus, serum is blood plasma without fibrinogen and other clotting factors; with proteins, other than fibrinogen, remaining in the serum. They are about equally divided between serum albumin and a great variety of serum globulins.

The serum proteins can be generally separated into serum albumin and serum globulins. Serum albumin, which is made in the liver, binds many small molecules for transport through the blood and helps maintain the osmotic pressure of the blood. The serum globulins are subdivided into alpha-globulins; beta-globulins; and gamma-globulins on the basis of their electrophoretic mobilities. Alpha globulins include, for example, the proteins that transport thyroxin and retinol (vitamin A). Alpha-2 macroglobulin, as will de discussed below, is known to bind, and thus to maintain in an inactive form, TGF-β.

Beta globulins include the iron-transporting protein transferrin. Gamma globulins contain most antibodies. The serum globulins also include the Complement system, which contains the main components of immune system activities, as well as other functional proteins and active peptides. The serum also contains serum lipids, including such basic components as Cholesterol (total), LDL cholesterol, HDL cholesterol, and Triglycerides.

The clotting factors contained within the plasma are critical to effective healing and are essential components of the extrinsic coagulation pathway. These factors function in harmony with factors in the platelets to effectively form the haemostatic plug and promote regeneration. In addition to plasma and serum proteins, increasing attention is being drawn to other plasma components that play crucial roles in coagulation, such as Mg²⁺ ions. Sekiya, F. et al., "Magnesium(II) is a Crucial Constituent of the Blood Coagulation Cascade," J. Biol. Chem. 271(15):8541-8544 (1996).

The extrinsic blood clotting pathway is initiated at the site of injury in response to the release of tissue factor (factor III). Tissue factor is a cofactor in the factor VIIa-catalyzed activation of factor X. Factor VIIa, a *gla* residue containing serine protease, cleaves factor X to factor Xa in a manner identical to that of factor IXa of the intrinsic pathway. The activation of factor VII occurs through the action of thrombin or factor Xa. The ability of factor Xa to activate factor VII creates a link between the intrinsic and extrinsic pathways. An additional link between the two pathways exists through the ability of tissue factor and factor VIIa to activate factor IX. The formation of complex between factor VIIa and tissue factor is believed to be a principal step in the overall clotting cascade.

In light of these and other reports, it would be advantageous to have a system, and the instant invention provides one, for activating platelets, without the need for foreign agonists like bovine thrombin, and that also provides the full range of useful compounds derived from platelet lysis, but absent certain compounds derived from a platelet release reaction, as well as preserving, in such a system, the regenerative factors found in the plasma.

### SUMMARY OF INVENTION

In its most general configuration, the present invention advances the state of the art with a variety of new capabilities and overcomes many of the shortcomings of prior devices in new and novel ways. In its most general sense, the present invention overcomes the shortcomings and limitations of the prior art in any of a number of generally effective configurations.

In one configuration, the present invention relates to a method for the production of a small amount of regenerative agent suitable for a single use. In an exemplar of this disclosure a 10 ml sample of blood is collected from an individual, allowed to clot, and the serum is separated by techniques well known in the art. This serum is reserved for later use in the preparation of the regenerative agent. A second 4.5 ml blood sample is placed in a tube containing buffered calcium citrate. The sample containing calcium citrate is centrifuged at 3,000 g for 30 minutes. The superior two thirds of the supernatant, which contains the plasma and platelets, or platelet rich plasma (PRP), is removed and the pellet containing red blood cells and leukocytes is discarded. The PRP is then combined with calcium chloride (CaCl₂) and a part of the serum reserved from the first blood sample. The calcium chloride induces massive platelet lysis, releasing the granular and other internally sequestered compounds and the platelet cytosolic components, and resulting in the formation of a clotted mass of platelet fragments, preferably after incubation at 37° C for 10 minutes. It is disclosed herein that for large scale production suitable for storage and use over a protracted period of time, platelet units of approximately 60 ml of platelet concentrate are obtained by standard blood processing methods, as would be know to one skilled in the art. Calcium chloride is added to each unit. The platelet concentrate with added calcium chloride is then gently mixed and incubated, preferably at 37° for 24 hours. In both the small scale and large scale methods detailed above, after incubation, a solid retracted white mass is left suspended free in a liquid phase in the container. The mass is then removed and the liquid phase transferred to a new container. In order to remove platelet membrane fragments that may remain, in one embodiment, the liquid phase is filtered through a 0.4 µm pore membrane. It is also disclosed herein that the liquid phase is centrifuged at 3,000 g for 1 hour. The final product may then be frozen or lyophilized for future use. The final agent may be mixed with at least one pharmaceutically acceptable excipient and may be used in the form of a cream, spray, or in any other form as would be obvious to one skilled in the art.

Investigation has shown that the process and compound of the instant invention, which is based on platelet lysis in a media of serum, yields a far different compositional profile than platelet activation in an aqueous media, as illustrated by the technique of Knighton ('938). Two dimensional gel electrophoresis of the agent prepared according to the instant invention shows a markedly different protein profile compared to the products of platelet activation with thrombin in an aqueous medium. Differences in protein expression over the prior art are also indicated by the results of gene array analysis.

Furthermore, the instant invention is a marked improvement over the prior art in its minimization of immunologic issues. Major roadblocks to the use of platelet containing or platelet derived products have been immunologic issues. Platelets are well known to carry HLA antigens on their surface. Platelet preparations, even those containing fragmentized platelets, carry these antigens into donors. This can be particularly problematic with repeated exposure to random platelets, leading to severe compatibility crises upon subsequent treatments.

Another major advance of the instant invention is that the lysis of platelets, followed by centrifugation and ultrafiltration, removes essentially all platelet membranes, including small fragments, and leaves behind non-immunogenic platelet proteins. Experimental evaluation of the instant invention compared to preparations of the prior art ('938) method showed this removal of potentially immunogenic fragments was accomplished with high efficiency. In skin testing with immune competence-screened human volunteers, the agent of the instant invention showed no immungenic activity, compared with reaction levels of between 6% and 10% for compounds prepared according to the prior art.

In a mouse model, the agent according to the instant invention showed improvements in skin elasticity and strength, after injury; and cellular proliferation, when compared with compounds prepared according to the prior art.

It is critical to differentiate the plurality of products normally found sequestered in platelet structures in the instant invention from the products that are released in the platelet release reactions, as typified by the Knighton process detailed above. This differentiation centers around the suppression of active TGF-β in the instant invention, compared with the large quantity of active TGF-β produced in the Knighton process. Suppression of active TGF-β in a wound healing adjunct such as that of the instant invention will tend to promote a healing process with less scarring that one containing active TGF-β.

When platelets are clotted with thrombin in the platelet release reaction, as in the Knighton process, the clotting process causes activation of the pro-TGF-β. This is particularly pronounced when the platelet clotting reaction occurs in the extremely acidic environment (pH = 6.5) proposed by Knighton. As a result, the materials released by platelets in the platelet release reaction by Knighton include the entirety of those compounds released by the platelets in the platelet release reaction, without any processing or other treatment that would affect the bioactivity of the multitude of factors contained therein, that is, with high levels of active TGF-β.

To the contrary, care is taken in the instant invention specifically to avoid the platelet release reaction and to exclude a certain component of the platelet release reaction as described by Knighton, that is, to suppress activation of TGF-β. This is accomplished in a number of novel ways, as will be detailed below, including the lysis of platelets with calcium and the addition to the composition of serum containing alpha-2 macroglobulin. In alternate embodiments, the composition may be further treated by adding compounds that block the physiologic effects of TGF-β; which may, be way of example only, be decorin; or it may be treated by actively extracting TGF-β from the composition, by way of example and not limitation, by means of passing the composition through an appropriate affinity column.

Thus, in sum, the composition of the instant invention is specifically produced and treated to comprise the compounds contained in platelet α-granules, dense bodies, and cytosol; but to be deficient in at least one of the materials released by platelets during the platelet release reaction: active TGF-β.

The composition of the instant invention, which is normally held at an approximately physiologic pH, may have the inactive TGF-β activated by, for example only, exposing it to an acidification process. This would create a composition with effects more resembling those of compositions containing materials released by platelets during the platelet release reaction, but with the additional benefits of providing compounds contained in the platelet α-granules, dense bodies, and cytosol.

In sum, the instant invention leads to a therapeutic agent that, compared to a compound formed by a process involving simply the activation and release reaction of platelets, gives a difference in the quantity and structure of regenerative products, including the suppression of active TGF-β. The instant invention also provides a vastly increased quantity of proteins compared to that of the platelet release reaction, and the avoidance of non-homologous thrombin in the final product.

### BRIEF DESCRIPTION OF THE DRAWINGS

Without limiting the scope of the present invention as claimed below and referring now to the drawings and figures:
FIG. 1 shows a two dimensional gel electrophoresis of a platelet release reaction product according to the prior art;
FIG. 2 shows a two dimensional gel electrophoresis of a preparation of the instant invention.
FIG. 3 shows a gene microarray analysis of up-regulated and down-regulated genes/pathways in skin cells comparing the instant invention with the prior art;
FIG. 4 shows a gene microarray analysis of up-regulated and down-regulated genes/pathways in endothelial cells comparing the instant invention with the prior art;
FIG. 5 shows a gene microarray analysis of up-regulated and down-regulated genes/pathways in fibroblast cells comparing the instant invention with the prior art;
FIG. 6 shows a gene microarray analysis of up-regulated and down-regulated genes/pathways in macrophage cells comparing the instant invention with the prior art;
FIG. 7 shows a skin elasticity study, following surgical trauma, in the mouse, comparing the agent of the instant invention with a control product;
FIG. 8 shows a skin strength study, following surgical trauma, in the mouse, comparing the agent of the instant invention with a control product; and
FIG. 9 shows a comparison of photomicrographs of mouse skin cell growth in isolated preparations, comparing the growth seen with minimal essential media supplemented with 10% serum (A) contrasted with the growth seen with minimal essential media supplemented with a 10% concentration of the agent of the instant invention (B).
FIG. 10 shows a schematic representation of the platelet release reaction and activation of TGF-β in the prior art, contrasted with the maintenance of inactive TGF-β in the instant invention.
FIG. 11 shows a schematic representation of the maintenance of inactive TGF-β in the instant invention, both by the mechanism of platelet lysis and the inactivation of TGF-β by binding with alpha-2 macroglobulin.

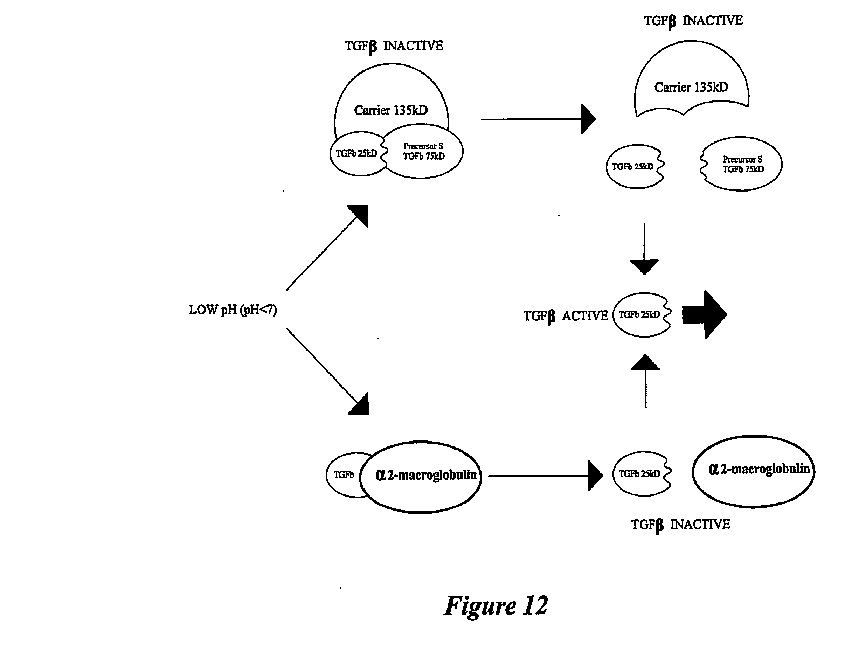

FIG. 12 shows a schematic representation of an alternative embodiment of the instant disclosure, in which TGF-β may be activated by, for example only, exposing it to an acidification process.

### DETAILED DESCRIPTION OF THE INVENTION

The method and materials of biological tissue repair of the instant invention enables a significant advance in the state of the art. The preferred embodiments of the method and materials accomplish this by new and novel arrangements of elements and methods that are configured in unique and novel ways and which demonstrate previously unavailable but preferred and desirable capabilities.

The detailed description set forth below in connection with the drawings is intended merely as a description of the presently preferred embodiments of the invention, and is not intended to represent the only form in which the present invention may be constructed or utilized. The description sets forth the designs, functions, means, and methods of implementing the invention in connection with the illustrated embodiments. It is to be understood, however, that the same or equivalent functions and features may be accomplished by different embodiments that are also intended to be encompassed within the spirit and scope of the invention.

It is disclosed that for the production of a small amount of regenerative agent suitable for a single use, a 10 ml sample of blood is collected from an individual, allowed to clot and the serum is separated by techniques well known in the art. This serum is reserved for later use in the preparation of the regenerative agent. A second 4.5 ml blood sample is placed in a tube containing 0.129 M (3.8%) buffered calcium citrate. The samples may be obtained simultaneously, by way of example and not limitation, by means of sequential blood draws through a single needle, as seen with the VACUTAINER® blood drawing system (Becton-Dickinson and Company, Franklin Lakes, NJ). The sample containing calcium citrate is centrifuged at 3,000 g for 30 minutes. The superior two thirds of the supernatant, which contains the plasma and platelets, or platelet rich plasma (PRP), is removed and the pellet containing red blood cells and leukocytes is discarded. The PRP is then combined with 100µl of 10% calcium chloride (CaCl₂) and 100 µl of the serum reserved from the first blood sample. The calcium chloride induces massive platelet lysis, releasing the granular and other internally sequestered compounds and the platelet cytosolic components, and resulting in the formation of a clotted mass of platelet fragments, preferably after incubation at 37° C for 10 minutes. It is also disclosed that for large scale production suitable for storage and use over a protracted period of time, platelet units of approximately 60 ml of platelet concentrate are obtained by standard blood processing methods, as would be known to one skilled in the art. Approximately 2.5 ml of a 10% calcium chloride solution are added to each unit. The platelet concentrate with added calcium chloride is then gently mixed and preferably incubated at 37° for 24 hours. A similar technique is followed as with the smaller scale formulations.

In both the small scale and large scale methods detailed above, after incubation, a solid retracted white mass is left suspended free in a liquid phase in the container. The mass is then removed and the liquid phase transferred to a new container. In order to remove platelet membrane fragments that may remain, in one embodiment the liquid phase is filtered through a 0.4 µm pore membrane. It is also disclosed that such fragments are removed by centrifuging the liquid phase at 4,000 rpm for 1 hour. This processing renders the agent, in one embodiment, non-immunogenic by skin testing. It is also disclosed that the agent further includes less than 0.1% w/w cell membrane fragments after processing.

The agent may then be frozen or lyophilized for future use, may be combined with at least one pharmaceutically approved excipient, and may be applied in the form of a liquid, cream, spray, or in any other form as would be known to one skilled in the art.

Investigation has shown that the process and compound of the instant invention, which is based on an agent of the compounds released by platelet lysis, in a media of plasma, yields a far different compositional profile than platelet activation in a aqueous media, as illustrated by the technique of Knighton ('938).

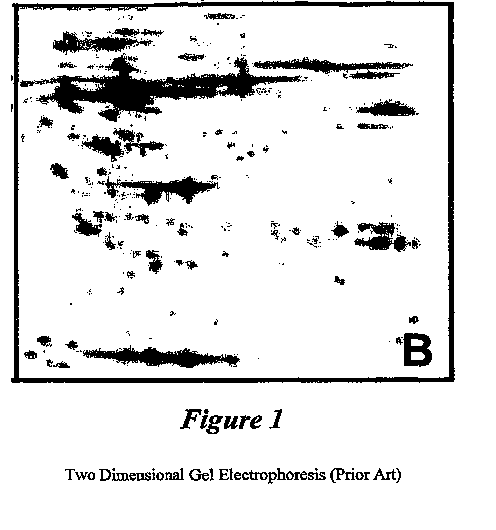

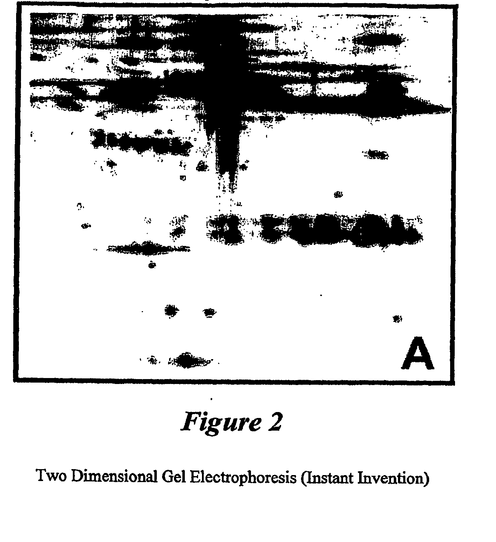

For example, two-dimensional gel electrophoresis of the instant invention compared to that of the platelet release compounds derived from the Knighton ('938) method show marked differences in protein composition, with a larger array of proteins in the instant invention, as compared to the prior art.. Figure 1 shows the two dimensional gel electrophoresis of washed platelet concentrate, activated with thrombin, and prepared according to the prior art ('938). In Figure 2, two dimensional gel electrophoresis of the compound of the instant invention is seen. Gels from both preparations were made and analyzed in an identical manner, as follows below.

Test preparations were centrifuged at 3000 g for 10 minutes. Then, a 1/10 volume of a solution containing Tris-HCl pH 8.0 (0.3 M), KCl (1.4 M) and MgCl2 (30 mM) was added to the supernatant and ultracentrifuged at 54000 g for 2 hours. The supernatant was diluted with 3 volumes of distilled water to decrease the salt concentration and then concentrated down to 30 µl in a MICROSEP^{™} brand centrifugal concentrator (Filtron, Northborough, MA). The concentrated protein sample was mixed and solubilized with 70 µl of a solution containing urea (8 M), CHAPS (4% w/v), Tris (40 mM), DTE (65 mM) and a trace of bromophenol blue. The final diluted sample was loaded on the first dimensional separation.

A non-linear immobilized pH gradient (3.5-10.0 NL IPG 18 cm) was used as the first dimension. It offered high resolution, excellent reproducibility and allowed high protein loads. Based on those specifications, the non-linear pH gradient strips were prepared by Pharmacia-Hoeffer Biotechnology AB and are commercially available. The strips were 3 mm wide and 180 mm long. The voltage was linearly increased from 300 to 3500 V during 3 hours, followed by 3 additional hours at 3500 V, whereupon the voltage was increased to 5000 V for an overnight run.

In the second dimension, a vertical gradient slab gel with the Laemmli-SDS-discontinuous system was used with some small modifications. Gels (160 x 200 x 1.5 mm size) were used, not polymerized in the presence of SDS, to prevent the formation of micelles of acrylamide monomer, thus increasing the homogeneity of pore size. The SDS used in the gel running buffer was sufficient to maintain the necessary negative charge on proteins. Piperazine diacrylyl (PDA) was used as cross-linker. Sodium thiosulfate was used as an additive to reduce background in the silver staining of gels. The second dimension running conditions included: Current: 40 mA/gel (constant); Voltage: 250 V; Temperature: 10° C constant; Time: 5 hours.

After the final run, gels were removed from the glass plates, washed in deionized water for 5 minutes, and transferred for staining to containers placed on orbital shakers operating at 36 rpm. A silver staining protocol consisted of treating the gels as follows: Gels were soaked in ethanol: acetic acid: water mixture (40:10:50) for 1 hour, then soaked in an ethanol: acetic acid: water mixture (5:5:90) for at least 2 hours. Gels were then washed in deionized water for 5 min in each wash and soaked in a solution containing glutaraldehyde (1%) and sodium acetate (0.5 M) for 30 min in each soak. The gels were then washed 3 times in deionized water for 10 min, soaked twice in a 2,7 naphtalene-disulfonic acid solution (0.05% w/v) for 30 min, and then rinsed 4 times in deionized water for 15 minutes in each rinse.

The gels were stained in a freshly made ammoniacal silver nitrate solution for 30 minutes. After staining, they were washed 4 times in deionized water for 4 min. in each wash. The gels were then developed in a solution containing citric acid (0.01% w/v) and formaldehyde (0.1% v/v) for 5 to 10 min. When a slight background stain appeared, development was stopped with a solution containing Tris (5% w/v) and acetic acid (2% v/v).

Comparison of the results of the above two-dimensional gel electrophoresis of the instant invention compared to that of the platelet release compounds derived from the prior art ('938) show marked differences in protein composition.

Comparison of Figures 1 (prior art) and 2 (instant invention) show that the instant invention, containing both cytosolic platelet proteins and plasma proteins, contains a far larger array of proteins than that prepared according to the prior art, which is a method of deriving compounds comprised primarily or exclusively of platelet granular material. This increased array of proteins in the final agent is consistent with observations that have identified at least 125 platelet cytosolic proteins. Marcus, K. et al., "Identification of Platelet Proteins Separated by Two-Dimensional Gel Electrophoresis and Analyzed by Matrix Assisted Laser Desorption/Ionization-Time of Flight-Mass Spectrometry and Detection of Tyrosine-Phosphorylated Proteins," Electrophoresis 21(13):2622-36 (2000).

An even more dramatic difference between the preparation of the instant invention and that of the prior art is seen with gene array analysis. Gene array analysis is a means of determining the relative transcript levels of a multitude of genes at a given point in time. The data inform the researcher which genes from among a subset of targets (or suspects) are actually being expressed in the mRNA library of the cells analyzed at a given snapshot in time. This data also shows the relative robustness of the expression based on the amount of target that is hybridized.

Such data is extremely useful to determine if a given treatment or condition has effected the mRNA expression profile, that is, to answer the question as to whether or not the treatment changed the gene expression profile. This information includes not only information as to which of the target genes are being expressed, but also provides information as to the level of expression, or intensity, of the gene expression. The level of mRNA often is reflective of the levels of corresponding protein. Therefore dramatic increases in mRNA are often accompanied by increases in protein expression.

Gene arrays are solid supports upon which a collection of gene-specific nucleic acids have been placed at defined locations, either by spotting or direct synthesis. In array analysis, a nucleic acid-containing sample is labeled and then allowed to hybridize with the gene-specific targets on the array. The nucleic acids attached to arrays are called "targets," whereas the labeled nucleic acids comprising the sample are called "probes." Based on the amount of probe hybridized to each target spot, information is gained about the specific nucleic acid composition of the sample. The major advantage of gene arrays is that they can provide information on thousands of targets in a single experiment. In this experiment the target sequences were a set of genes known to be involved in the wound healing profile.

A typical gene array experiment involves: 1) isolating RNA from the samples to be compared; 2) converting the RNA samples to labeled cDNA via reverse transcription, and this step may be combined with aRNA amplification; 3) hybridizing the labeled cDNA to identical membrane or glass slide arrays; 4) removing unhybridized cDNA; 5) detecting and quantifying the hybridized cDNA; and 6) comparing the quantitative data from the various samples.

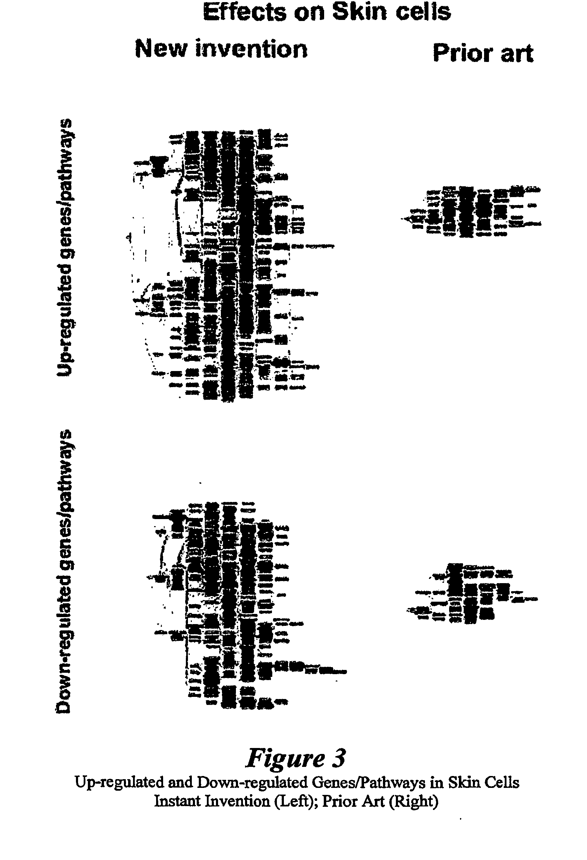

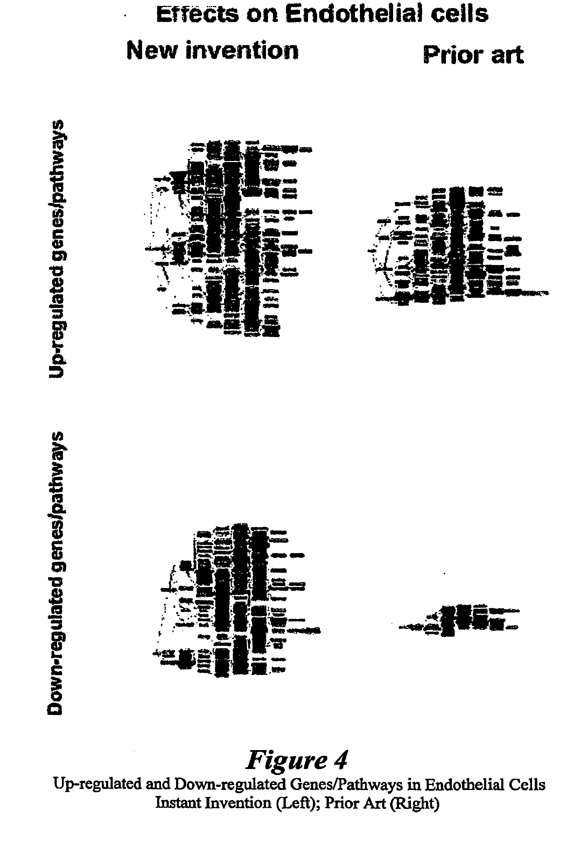

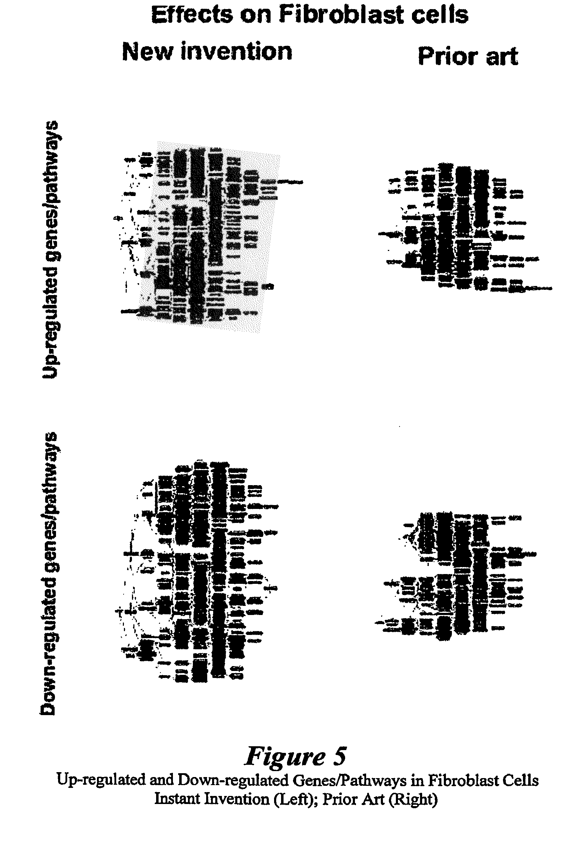

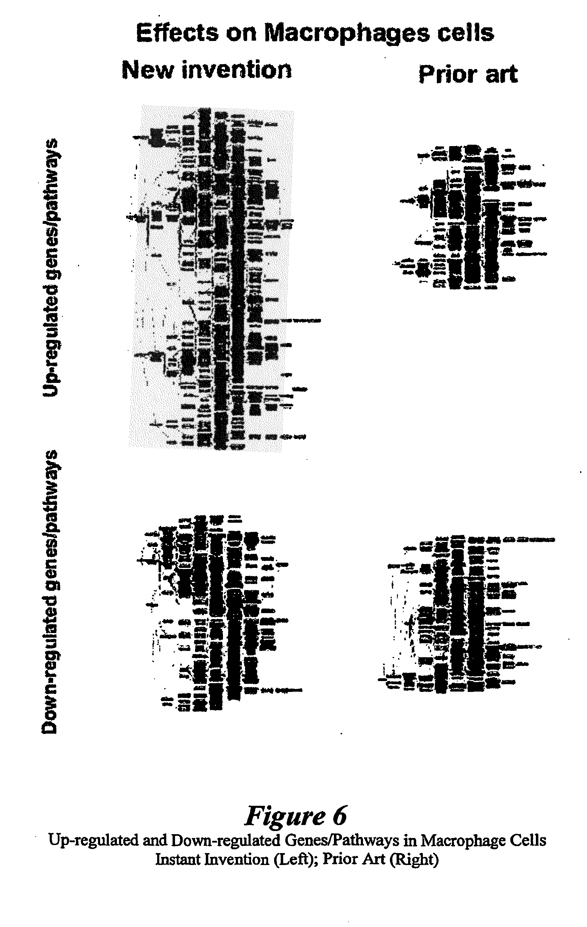

Products that result from the method of isolating compounds from the platelet activation process, according to the method of the '938 patent, were compared with the products that result from the instant invention in terms of their effects on the gene expression profiles of four cell types relevant to wound healing. The technique of gene microarray analysis was performed to compare gene expression levels in cells treated with compounds derived from the '938 process, with those of the instant invention. These studies indicate a sharp contrast in mRNA expression upon treatment with the two different formulas. As seen in Figs. 3-6, gene array analysis shows a marked increase in mRNA expression in skin cells, endothelial cells, fibroblasts, and macrophages, between the instant invention (left in all figures) and the prior art (right in all figures).

The results clearly demonstrate that the instant invention induces a far more dramatic mRNA expression profile based on the targets evaluated. This finding suggest that 1) platelet derived factors rendered from calcium induced platelet lysis have a more dramatic and robust effect on the cells relevant to healing and 2) the factors derived from calcium induced lysis are not the same as those produced from the platelet release reaction. Furthermore, analysis suggests that the expression difference is likely due to both an increase in the quantity of the same factors that occur in the '938 invention and also from novel factors that only are seen using the instant invention, i.e., the factors that are now available in the retained serum, the platelet cytoplasm, and from the more complete granule emptying that occurs with platelet lysis.

Prior to the instant invention, major roadblocks to the use of platelet containing or platelet derived products have been immunologic issues. Platelets are well known to carry HLA antigens on their surface. Platelet preparations, even those containing fragmentized platelets, carry these antigens into donors. This can be particularly problematic with repeated exposure to random platelets, leading to severe compatibility crises upon subsequent treatments.

Another major advance of the instant invention is that the lysis of platelets, followed by centrifugation and ultrafiltration, removes essentially all platelet membranes, including small fragments, and leaves behind non-immunogenic platelet proteins. Experimental immunoreactivity evaluation of the instant invention compared to preparations of the prior art ('938) method showed this removal of potentially immunogenic fragments with high efficiency.

Thirty human volunteers were skin tested according to a standard skin scratch test protocol with both the instant invention, that is, the ultrapurified platelet and serum product of the instant invention, and also with the compound formulated according to the prior art, which is believed to retain some platelet membrane fragments. Volunteers were examined for both immediate reaction to the two compounds, and for delayed hypersensitivity upon retesting 30 days after the initial contact. All volunteers were internally controlled using a control skin test with histamine, to insure reactivity of all volunteers. The results are shown in Table A:

**Table A: Skin Testing for Immunogenic Components; Instant Invention and Prior Art**

| | Instant Invention | Histamine Control | Prior Art | Histamine Control |
|---|---|---|---|---|
| Positive skin test reactions at time = 1 day | 0/33 (0%) | 33/33 (100%) | 3/33 (10%) | 33/33 (100%) |
| Positive skin test reactions at time = 30 day | 0/33 (0%) | 33/33 (100%) | 2/33 (6%) | 33/33 (100%) |

The instant invention showed no immunologic reaction on skin testing, either with initial exposure or with a second challenge at 30 days. In contrast, the compound prepared according to the prior art ('938) method showed an initial reaction rate of 10% and a delayed hypersensitivity reaction of 6% with a second challenge at 30 days. As repeated exposure to antigens may lead to heightened immunogenic response, it is inferred that increasing numbers of hypersensitivity reactions would be seen with additional exposures to the prior art products.

Additionally, animal experiments were undertaken to evaluate the efficacy of the instant invention for the promotion of tissue regeneration. In the first experiment, skin healing was evaluated in a mouse model.

Fifty mice were anesthetized with intramuscular ketamine. The skin hair was removed with clippers from an area slightly longer than 10 mm on the back of each. An incision was made 10 mm long in the denuded area to a depth down to the muscle aponeurosis. The wound was disinfected by swabbing with hydrogen peroxide and hemorrhage was contained with manual compression for two minutes. A preparation containing the instant invention was applied to the surface of the wound. A second application of the preparation of the instant invention was reapplied 24 hours later.

A control group of twenty mice was simultaneously prepared according to the same surgical protocol. In this group, the surgical wound was treated with an application of mouse serum with PDGF in a concentration of 50 pg/ml, to imitate the common clinical protocol of Nagai *et al.,* based on the use of recombinant PDGF (REGRANEX™ brand recombinant PDGF from Ethicon, Inc. of Somerville, N.J.) for wound healing, followed by a second application of the same compound 24 hours later. Therefore, the active and control groups differed in the active group receiving a compound with the platelet cytosolic proteins derived according to the instant invention, as well as plasma constituents, while the control group received only plasma constituents and PDGF. Therefore, differences observed between the groups are believed to be derived from the presence of platelet cytosolic proteins in the treatment.

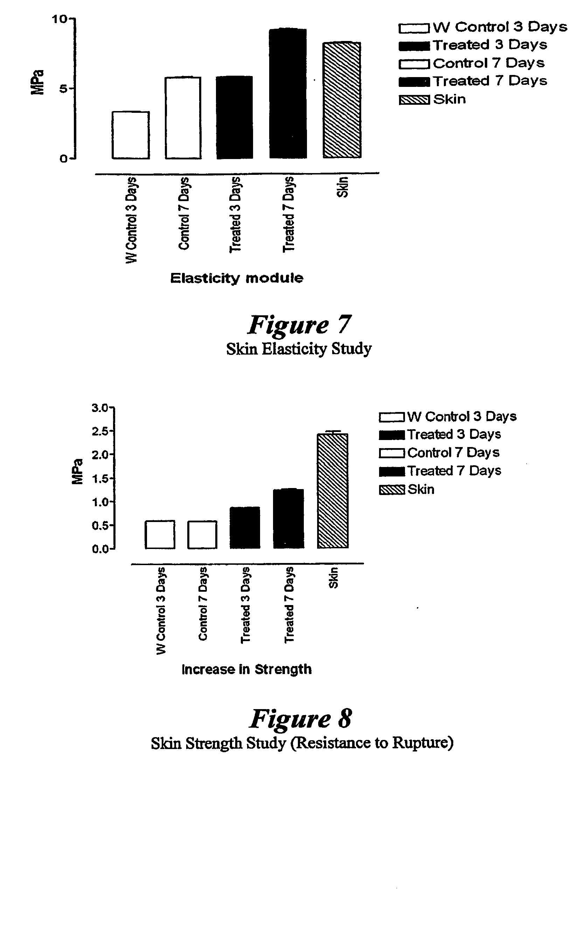

Twenty five mice from the active group and ten mice from the control group were sacrificed on the third day post-treatment. A square of skin was removed, leaving the previous surgical incision in the center of the square. The skin was transferred to a universal traction assay system (MIDI 5-5, Messphisik, Germany). This device allows the measurement of the elasticity of the skin, as well as traction to the point of rupture, which enabled measurements to be made of the relative amount of force necessary to disrupt the healing tissues, as described by Hara *et al.* and Draaijers *et al.* The results are shown below in FIGS. 7 and 8.

In the elasticity study, seen in FIG. 7, the elasticity of the skin in the control group, that is, the group receiving mouse serum and PDGF as a topical treatment, showed a marked diminution over normal skin values (cross hatched area; far right) three days after injury. This was somewhat improved seven days after injury, but still remained considerably below normal levels. In contrast, in the mice treated with the preparation according to the instant invention, there was less of a decrease from normal values three days after injury, and skin elasticity had returned to normal levels, or even to improved levels of elasticity, seven days after injury, compared to baseline values.

In the skin strength, or resistance to rupture study, seen in FIG. 8, there was a dramatic post-injury reduction in the resistance of the skin to rupture under traction in those mice treated with the control preparation, that is, mouse serum and PDGF as a topical treatment, to less than 25% of the pre-injury tissue strength (cross-hatched area; far right). This was essentially unimproved, in the control group, seven days after injury. On the other hand, in mice treated with the preparation of the instant invention, the fall in rupture resistance was somewhat attenuated, compared to the controls, after three days; and by seven days had recovered to nearly half of the pre-injury level. The resistance to rupture of skin during healing is an important parameter, as it may be analogized to wound dehiscence following injury or surgery.

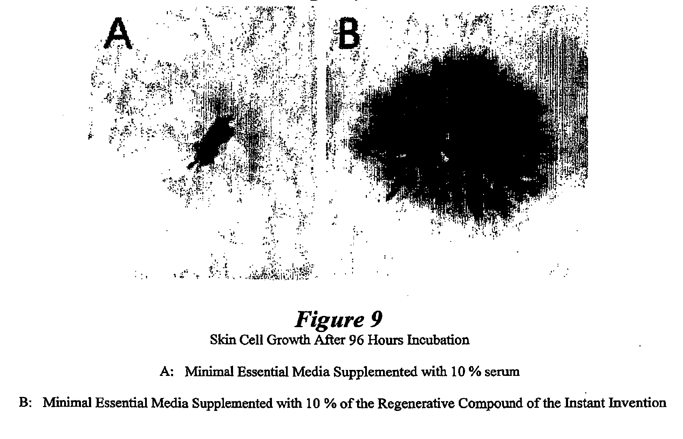

The method and agent of the instant invention is not only suitable for promoting wound healing. In an alternative embodiment, the salutary effect of the agent on cell growth may be used to promote transplantation, by mixing the agent with cells to be transplanted prior to applying the agent to the target tissue. By way of example and not limitation in such regard, experiments were undertaken to compare the relative rates of cell growth in isolated preparations bathed in either serum or the preparation of the instant invention (FIG. 9). Twenty fragments of mouse skin, each measuring 1 x 0.5 x 0.3 mm, were seeded on the surface of a tissue culture flask and covered with Dulbecco's Minimum Essential Medium, supplemented with 10% mouse serum. After 96 hours, the resultant cell growth, as viewed with a 3X lens, appears in FIG. 9A. At the same time, twenty fragments of mouse skin, each measuring 1 x 0.5 x 0.3 mm, were seeded on the surface of a tissue culture flask and covered with Dulbecco's Minimum Essential Medium, supplemented with 10% of the preparation of the instant invention, that is, serum and platelet cytosolic extracts. After 96 hours, the resultant cell growth, as viewed with a 3X lens, appears in FIG. 9B. The much higher rate of cell growth seen with the instant invention strongly supports the agent of the present invention as an adjunct to the transplantation of cells.

It is critical to differentiate the plurality of products normally found sequestered in platelet structures in the instant invention from the products that are released in the platelet release reactions, as typified by the Knighton process detailed above. This differentiation centers around the suppression of active TGF-β in the instant invention, compared with the large quantity of active TGF-β produced in the Knighton process.

Platelet transported TGF-β is a problematic derivative of platelets. Transforming growth factor-b (TGF-β) is the main cytokine causing fibroblast collagen deposition, as described by Tietjen and Stover. As discussed by Flanders et al., the excessive expression of TGF-β as been implicated as being responsible for accumulation of detrimental scar tissue. Therefore, suppression of active TGF-β in a wound healing adjunct such as that of the instant invention will tend to promote a healing process with less scarring that one containing active TGF-β.

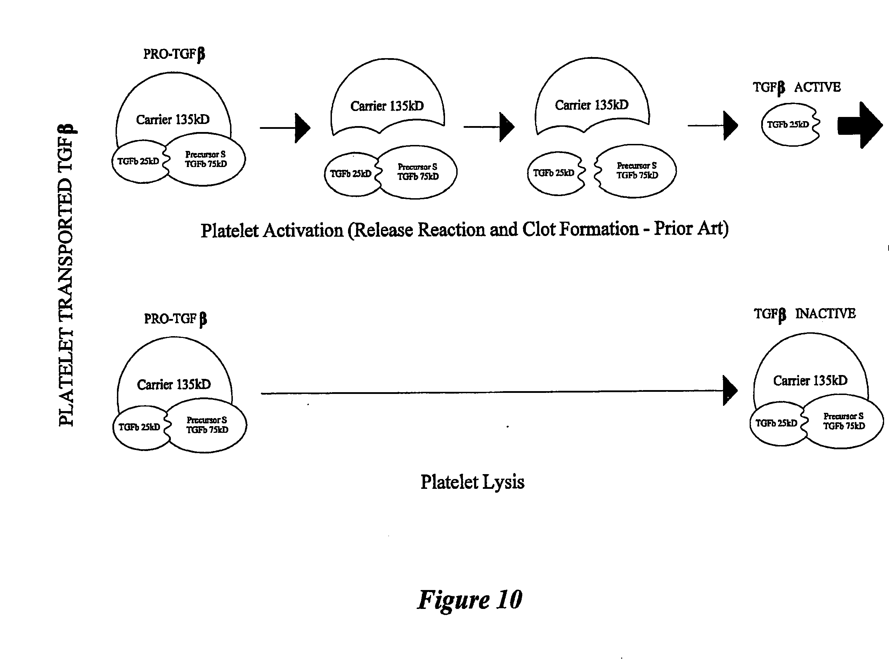

Platelet transported TGF-β is carried within platelet granules, as seen in Fig. 10, in an inactive form which may be called pro-TGF-β. Such pro-TGF-β consists of an approximately 25 kD (kilo Dalton) portion (mature TGF beta-1 dimer) and a 75 kD fragment of Precursor S; with both fragments bound to a 135 kD carrier. Grainger et al. denominates the Precursor S as the latent TGF-β binding protein or LTBP, and the 75 kD fragment of Precursor S as the latency-associated peptide or LAP, and states that over 95% of all TGF-β assayed in whole platelets is transported as this pro- TGF-β.

When platelets are clotted with thrombin in the platelet release reaction, as in the Knighton process, the clotting process causes activation of the pro-TGF-β, as discussed by Wakefield et al., which may in part be due to cleavage of the 25 kD (active) portion from the 75 kDa Precursor S and the 135 kDa carrier, as seen in Fig. 10. This is particularly pronounced when the platelet clotting reaction occurs in the extremely acidic environment (pH = 6.5) proposed by Knighton. As a result, the materials released by platelets in the platelet release reaction by Knighton include the entirety of those compounds released by the platelets in the platelet release reaction, without any processing or other treatment that would affect the bioactivity of the multitude of factors contained therein, that is, with high levels of active TGF-β.

To the contrary, care is taken in the instant invention specifically to avoid the platelet release reaction and to exclude a certain component of the platelet release reaction as described by Knighton, that is, to suppress activation of TGF-β. This is accomplished in a number of novel ways.

Firstly, the blood sample from which the compound of the instant invention is derived is divided into two samples; and the first is allowed to clot. This clotting of whole blood does not result in activation of the platelet release reaction, and, after removal of the clot, a serum remains that is essentially free of fibrinogen, thromboplastin, and platelets. The serum is, however, rich in alpha-2 macroglobulin, an important factor that will be discussed presently.

The second sample of blood is anticoagulated and centrifuged to form a platelet rich plasma containing both platelets and alpha-2 macroglobulin. When the serum from the first blood sample is combined with the platelet rich plasma from the second sample in the presence of calcium, the platelets lyse without undergoing activation of the platelet release reaction, releasing the inactive TGF-β, as well as the α-granule, dense body, and cytosolic platelet compounds.

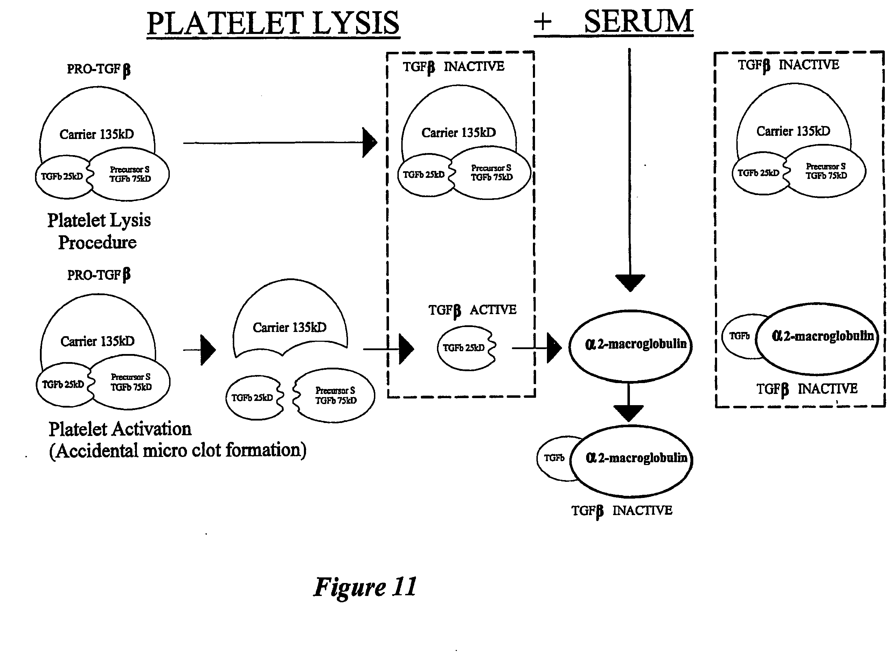

The presence of alpha-2 macroglobulin is important because, as discussed by O'Connor-McCourt and Wakefield, it is a potent binding agent of TGF-β, maintaining TGF-β in its inactive form. Thus, the alpha-2 macroglobulin present in the instant invention maintains the TGF-β in the instant invention in its bound, and inactive form, as seen in Fig. 11. Thus the composition of the instant invention contains inactive TGF-β, resulting from platelet lysis, and alpha-2 macroglobulin derived from serum, which effectively scavenges activated TGF-β that may inadvertently form, for example, by accidental micro-clot formation, or by activation due to an acidic environment at the site of application, as also seen in Fig. 11. In alternate embodiments, the composition may be further treated by adding compounds that block the physiologic effects of TGF-β, such as, by way of example, decorin; or it may be treated by extracting TGF-β from the composition, by way of example and not limitation, by means of passing the composition through an appropriate affinity column.

This may be contrasted with a composition containing the materials released by platelets during the platelet release reaction, as typified by the process of Knighton. The platelets are washed, resuspended in an acid buffer (ph=about 6.5), and the platelet release reaction is activated. The loss of the TGF-β binding alpha-2 macroglobulin by washing; the activation of the platelet release reaction by thrombin; and the highly acid environment, known from Wakefield et al. to be an activator of TGF-β; all combine to form active TGF-β. Thus, in sum, the composition of the instant invention is specifically produced and treated to comprise the compounds contained in platelet α-granules, dense bodies, and cytosol; but to be deficient in at least one of the materials released by platelets during the platelet release reaction: active TGF-β.

The suppression of active TGF-β is indicated in the experimental results seen with the composition of the instant invention. As seen in Fig. 7, skin elasticity after injury and seven days treatment is much greater than PDGF treated controls, and is actually greater in the experimental subjects than in the normal skin controls. At the same time, as seen in Fig. 8, skin strength in the experimental subjects is greater than that of the PDGF created controls, but is only about half that of normal skin controls after seven days treatment. This suggests a visible reduction in fibroblast over-stimulation, due to the suppression of TGF-β, in the subjects treated with the composition of the instant invention. This stands in contrast to the increased fibroblastic activity seen with compositions containing materials released by platelets during the platelet release reaction, for example, the compound of Knighton.

The suppression of TGF-β in the instant invention may make for a somewhat more elastic wound, at least in the early stages of healing, compared with that obtained with compositions containing materials released by platelets during the platelet release reaction, and it also confers advantages, such as decreased scarring. This may be advantageous particularly for applications in which scarring may be critical, such as, by way of example and not limitation, for corneal repairs or in plastic surgery.

It is also disclosed that the composition of the instant invention, which is normally held at physiologic pH, may have the inactive TGF-β activated by exposing it to an acidification process, for example only, by bubbling the composition with carbon dioxide to cleave the active 25 kD TGF-β from Precursor S and the carrier; as well as to cause dissociation of active 25 kD TGF-β from alpha-2 macroglobulin. This would create a composition with effects more resembling those of compositions containing materials released by platelets during the platelet release reaction, but with the additional benefits of providing compounds contained in the platelet α-granules, dense bodies, and cytosol.

It is to be emphasized that the preceding experiments relating to improvements in skin elasticity and strength, or to cell growth, are meant as illustrations and not limitations as to the agent, method, and process of preparation. The agent in general promotes tissues plasticity in a more general sense, that is, improvements in one or more parameters such as size, strength, cellular or organ function, matrix composition, or other physiologic measurements, as would be known to one skilled in the art. Additionally, the agent promotes angiogenesis and is substantially non-immunogenic.

What is claimed then, is a biological tissue regenerative agent obtainable by the claimed process comprising the materials released by platelet lysis, comprising compounds normally contained in platelet α-granules, dense bodies, and cytosol and wherein TGF-β is in an inactive form; and serum containing alpha-2 macroglobulin, for facilitating tissue growth, which may be more broadly described as including improvements in tissue plasticity. A process for preparing this agent, which may be for any animal or human use, may further be derived from the platelets and serum of the individual being treated, or from another individual. The agent may be substantially non-immunogenic and may contain less than 0.1% w/w cell membrane fragments, which may be removed from the agent by filtration, centrifugation, or any other technique as would be obvious to one skilled in the art.

The process for producing a biological tissue regenerative agent comprising the steps of:
(i) allowing a first sample of whole blood from an individual to clot and separate into clot and serum, discarding the clot, and reserving the serum;
(ii) anticoagulating a second sample of whole blood from said individual with an anticoagulant;
(iii) separating said second sample into
   a cellular fraction containing primarily red blood cells and leukocytes, and
   a platelet rich plasma fraction comprising a plurality of platelets, and discarding the cellular fraction;
(iv) subjecting said platelet rich plasma fraction of the second sample to a treatment to induce lysis of the platelets in said platelet rich plasma fraction of the second sample to form a platelet rich plasma fraction comprising lysed platelets and platelet structural fragments, said treatment comprising the addition of calcium to said platelet rich plasma fraction of the second sample;
(v) combining said platelet rich plasma fraction comprising lysed platelets and platelet structural fragments, with the serum of said first sample containing alpha-2 macroglobulin, to form a mixture; and
(vi) removing all of the platelet structural fragments from the mixture to render a biological tissue regenerative agent comprising the materials released by platelet lysis, wherein TGF-β is in an inactive form

It is disclosed that the ratio of the volume of the first sample and the second sample is 2:1. Processing of various components during the preparation may be improved or accelerated by centrifugation at predetermined speeds and for predetermined times, as well as by incubation at predetermined temperatures for predetermined times. In a preferred embodiment, the anticoagulant is buffered calcium citrate. It is also disclosed that platelet lysis, which may also be produced by freezing and thawing the platelets through a predetermined number of freeze-thaw cycles or by sonication, is induced by providing an amount of calcium, which may be in the form of calcium chloride. The agent may be purified by means such as filtration and centrifugation, and may be processed for storage by such means as, by way of example and not limitation, freezing and freeze-drying. The agent may be combined with at least one pharmaceutically approved excipient, and may be applied in the form of a liquid, cream, spray, or in any other form as would be known to one skilled in the art.

The method facilitates tissue growth, which may be more broadly described as including improvements in tissue plasticity, and may be applied to animal tissue, including mammalian and human tissue. The plurality of materials released by platelet lysis may be released from mammalian, including human, platelets, and may be derived from the individual whose tissue is being treated or from another individual. The treatment promotes tissue plasticity in at least one tissue, which may include, but is not limited to, such measurable parameters as improvements in skin elasticity and strength following injury; as well as to cell growth, including cell growth of transplanted cells. The method may involve application of the agent in any of various manners as would be known to one skilled in the art, including without limitation, topical application and application into a closed space. The increase in concentration and variety of compounds contained in the instant invention renders the method more therapeutically effective than a compound containing those released by a platelet releaser reaction.

In additional embodiments, compounds that block the physiologic effects of TGF-β, which may by way of example only, be decorin, may be added to the mixture; and a further step of removing TGF-β from the mixture maybe added, again by way of example only, by passing the mixture through an appropriate affinity column. It is also disclosed that inactive TGF-β in the mixture may be activated, by way of example only, by acidifying the mixture.

### INDUSTRIAL APPLICABILITY

The application answers a long felt need for a biological tissue regenerative agent and method for preparing the same. The agent and method of use provides for a plurality of compounds released by platelet lysis, further comprising compounds normally contained in platelet α-granules, dense bodies, and cytosol; and serum. The agent and method suppresses at lest one compound released by the platelet release reaction of the prior art; active TGF-β.

## Claims

1. A process for producing a biological tissue regenerative agent comprising the steps of:
(i) allowing a first sample of whole blood from an individual to clot and separate into clot and serum, discarding the clot, and reserving the serum;
(ii) anticoagulating a second sample of whole blood from said individual with an anticoagulant;
(iii) separating said second sample into
a cellular fraction containing primarily red blood cells and leukocytes, and
a platelet rich plasma fraction comprising a plurality of platelets, and discarding the cellular fraction;
(iv) subjecting said platelet rich plasma fraction of the second sample to a treatment to induce lysis of the platelets in said platelet rich plasma fraction of the second sample to form a platelet rich plasma fraction comprising lysed platelets and platelet structural fragments, said treatment comprising the addition of calcium to said platelet rich plasma fraction of the second sample;
(v) combining said platelet rich plasma fraction comprising lysed platelets and platelet structural fragments, with the serum of said first sample containing alpha-2 macroglobulin, to form a mixture; and
(vi) removing all of the platelet structural fragments from the mixture to render a biological tissue regenerative agent comprising the materials released by platelet lysis, wherein TGF-β is in an inactive form.

2. The process according to claim 1, wherein the anticoagulant is buffered calcium citrate.

3. The process according to claim 1, wherein the calcium is provided in the form of calcium chloride.

4. The process according to claim 1, wherein products normally found sequestered in platelet structures comprise products found sequestered in the alpha granules, and dense bodies of an intact platelet.

5. The process according to claim 1, wherein the step of removing all of the platelet structural fragments from the mixture further comprises filtration through a membrane with pores less than or equal to 0.4 µm.

6. The process according to claim 1, further comprising the step of removing TGF-β from said mixture.

7. The process according to claim 6, wherein the step of removing TGF-β from the agent is accomplished by adding decorin.

8. The process according to claim 6, wherein the step of removing TGF-β from the agent is accomplished by passing said mixture through an appropriate affinity column.

9. A biological tissue regenerative agent for facilitating tissue growth obtained according to the process of any of claims 1 to 8.

10. A biological tissue regenerative agent according to claim 9 for use in promotion of tissue regeneration.

11. A biological tissue regenerative agent according to claim 9 for use in promotion of wound healing.

12. A biological tissue regenerative agent according to claim 9 for use in promotion of transplantation.

13. A pharmaceutical composition comprising a biological tissue regenerative agent according to claim 9 and a pharmaceutically approved excipient.

## Patentansprüche

1. Verfahren zur Herstellung eines Regenerationsmittels für biologisches Gewebe, umfassend die Schritte:
(i) Gerinnenlassen einer ersten Vollblutprobe von einem Individuum und Auftrennenlassen derselben in Koagulat und Serum, Verwerfen des Koagulats und Aufbewahren des Serums;
(ii) Hemmen der Gerinnung einer zweiten Vollblutprobe von dem Individuum mit einem Antikoagulans;
(iii) Auftrennen der zweiten Probe in
eine Zellfraktion, die vorwiegend Erythrocyten und Leukocyten enthält; und
eine thrombocytenreiche Plasmafraktion, die eine Vielzahl von Thrombocyten umfasst; und
Verwerfen der Zellfraktion;
(iv) Behandeln der thrombocytenreichen Plasmafraktion der zweiten Probe zur Induktion einer Lyse der Thrombocyten in der thrombocytenreichen Plasmafraktion der zweiten Probe unter Bildung einer thrombocytenreichen Plasmafraktion, die lysierte Thrombocyten und Thrombocyten-Strukturfragmente umfasst, wobei die Behandlung die Zugabe von Calcium zu der thrombocytenreichen Plasmafraktion der zweiten Probe umfasst;
(v) Vereinigen der thrombocytenreichen Plasmafraktion, die lysierte Thrombocyten und Thrombocyten-Strukturfragmente umfasst, mit dem Serum der ersten Probe, das alpha-2-Makroglobulin enthält, unter Bildung eines Gemischs; und
(vi) Entfernen aller Thrombocyten-Strukturfragmente aus dem Gemisch, wobei man ein Regenerationsmittel für biologisches Gewebe gewinnt, das die durch eine Lyse von Thrombocyten freigesetzten Stoffe umfasst, wobei TGF-β in einer inaktiven Form vorliegt.

2. Verfahren gemäß Anspruch 1, wobei es sich bei dem Antikoagulans um gepuffertes Calciumcitrat handelt.

3. Verfahren gemäß Anspruch 1, wobei das Calcium in Form von Calciumchlorid bereitgestellt wird.

4. Verfahren gemäß Anspruch 1, wobei Produkte, die man in Thrombocytenstrukturen normalerweise maskiert vorfindet, Produkte umfassen, die man in den Alpha-Vesikeln und elektrondicthte-Vesikeln eines intakten Thrombocyten maskiert vorfindet.

5. Verfahren gemäß Anspruch 1, wobei der Schritt des Entfernens aller Thrombocyten-Strukturfragmente aus dem Gemisch weiterhin die Filtration durch eine Membran mit einer Porengröße von kleiner oder gleich 0,4 µm umfasst.

6. Verfahren gemäß Anspruch 1, das weiterhin den Schritt des Entfernens von TGF-β aus dem Gemisch umfasst.

7. Verfahren gemäß Anspruch 6, wobei der Schritt des Entfernens von TGF-β aus dem Mittel durch Zugabe von Decorin bewerkstelligt wird.

8. Verfahren gemäß Anspruch 6, wobei der Schritt des Entfernens von TGF-β aus dem Mittel dadurch bewerkstelligt wird, dass man das Gemisch durch eine geeignete Affinitätssäule leitet.

9. Regenerationsmittel für biologisches Gewebe zur Erleichterung des Gewebewachstums, erhalten nach dem Verfahren gemäß einem der Ansprüche 1 bis 8.

10. Regenerationsmittel für biologisches Gewebe gemäß Anspruch 9 zur Verwendung bei der Förderung der Geweberegeneration.

11. Regenerationsmittel für biologisches Gewebe gemäß Anspruch 9 zur Verwendung bei der Förderung der Wundheilung.

12. Regenerationsmittel für biologisches Gewebe gemäß Anspruch 9 zur Verwendung bei der Förderung einer Transplantation.

13. Pharmazeutische Zusammensetzung, die ein Regenerationsmittel für biologisches Gewebe gemäß Anspruch 9 und einen pharmazeutisch zugelassenen Arzneimittelhilfsstoff umfasst.

## Revendications

1. Procédé de production d'un agent de régénération de tissu biologique, comprenant les étapes suivantes :
(i) laisser un premier échantillon de sang entier provenant d'un individu coaguler et se séparer en caillot et en sérum, éliminer le caillot et conserver sérum ;
(ii) provoquer l'anticoagulation d'un second échantillon de sang entier provenant dudit individu avec un anticoagulant ;
(iii) séparer ledit second échantillon en
une fraction cellulaire contenant principalement des globules rouges et des leucocytes, et
une fraction de plasma riche en plaquettes comprenant une pluralité de plaquettes, et éliminer la fraction cellulaire ;
(iv) soumettre ladite fraction de plasma riche en plaquettes du second échantillon à un traitement pour induire la lyse des plaquettes dans ladite fraction de plasma riche en plaquettes du second échantillon pour former une fraction de plasma riche en plaquettes comprenant des plaquettes lysées et des fragments structurels de plaquette, ledit traitement comprenant l'ajout de calcium à ladite fraction de plasma riche en plaquettes du second échantillon ;
(v) combiner ladite fraction de plasma riche en plaquettes comprenant des plaquettes lysées et des fragments structurels de plaquette avec le sérum dudit premier échantillon contenant de l'alpha-2 macroglobuline pour former un mélange ; et
(vi) éliminer tous les fragments structurels de plaquette du mélange pour donner un agent de régénération de tissu biologique comprenant les substances libérées par la lyse des plaquettes, dans lequel le TGF-β est sous une forme inactive.

2. Procédé selon la revendication 1, dans lequel l'anticoagulant est du citrate de calcium.

3. Procédé selon la revendication 1, dans lequel le calcium est fourni sous la forme de chlorure de calcium.

4. Procédé selon la revendication 1, dans lequel les produits que l'on trouve normalement séquestrés dans les structures des plaquettes comprennent des produits que l'on trouve séquestrés dans les granules alpha, et les corps denses d'une plaquette intacte.

5. Procédé selon la revendication 1, dans lequel l'étape d'élimination de tous les fragments structurels de plaquette du mélange comprend en outre une filtration à travers une membrane ayant des pores inférieurs ou égaux à 0,4 µm.

6. Procédé selon la revendication 1, comprenant en outre l'étape d'élimination du TGF-β dudit mélange.

7. Procédé selon la revendication 6, dans lequel l'étape d'élimination du TGF-β de l'agent est réalisée par l'ajout de décorine.

8. Procédé selon la revendication 6, dans lequel l'étape d'élimination du TGF-β de l'agent est réalisée en passant ledit mélange à travers une colonne d'affinité appropriée.

9. Agent de régénération de tissu biologique servant à faciliter la croissance tissulaire, obtenu selon le procédé de l'une quelconque des revendications 1 à 8.

10. Agent de régénération de tissu biologique selon la revendication 9, pour son utilisation dans la régénération d'un tissu.

11. Agent de régénération de tissu biologique selon la revendication 9, pour son utilisation dans la cicatrisation.

12. Agent de régénération de tissu biologique selon la revendication 9, pour son utilisation pour favoriser une transplantation.

13. Composition pharmaceutique, comprenant un agent de régénération de tissu biologique selon la revendication 9 et un excipient acceptable en pharmacie.
